# FASCICULE DE BREVET EUROPEEN

(11) **EP 3 313 313 B1**
(45) Date de publication et mention de la délivrance du brevet: **27.01.2021**
(21) Numéro de dépôt: 16733416.8
(22) Date de dépôt: 24.06.2016
(51) Int. Cl.: A61B 34/10, G06T 7/00

(54) **SYSTÈME D'AIDE AU GUIDAGE D'UN OUTIL ENDOVASCULAIRE DANS DES STRUCTURES VASCULAIRES**
SYSTEM ZUR FÜHRUNG EINES ENDOVASKULÄREN INSTRUMENTS IN GEFÄSSSTRUKTUREN
SYSTEM FOR HELPING TO GUIDE AN ENDOVASCULAR TOOL IN VASCULAR STRUCTURES

(30) Priorité: 26.06.2015 FR 1555944
(43) Date de publication de la demande: 02.05.2018
(73) Titulaire: THERENVA, 35000 Rennes (FR)
(72) Inventeur: DUMENIL, Aurélien, 02700 Tergnier (FR); GÖKSU, Cemil, 31400 Toulouse (FR); LALYS, Florent, 35700 Rennes (FR); LUCAS, Antoine, 35690 Acigné (FR)
(74) Mandataire: Vidon Brevets & Stratégie
(86) Numéro de dépôt international: PCT/EP2016/064681
(87) Numéro de publication internationale: WO 2016/207358

(56) Documents cités:
- WO-A1-2011/128797
- WO-A2-03/088143
- WO-A2-2008/087629
- US-A1- 2005 004 454
- US-A1- 2009 088 830
- US-A1- 2013 303 893

## Description

La présente invention concerne un système d'aide au guidage d'un outil endovasculaire dans des structures vasculaires, et un produit programme d'ordinateur associé.

Elle trouve une application dans le domaine des interventions endovasculaires guidées par l'image.

Les interventions endovasculaires permettent de traiter des maladies vasculaires de façon minimalement invasive. Elles consistent généralement à insérer par voie endovasculaire un dispositif médical dans le but d'interagir avec les tissus pathologiques. Des interventions endovasculaires sont notamment utilisées pour traiter les anévrismes aortiques ainsi que les sténoses et les thromboses artérielles, via l'introduction de divers d'outils endovasculaires adaptés tels qu'un ballon ou un stent.

Contrairement aux interventions chirurgicales classiques qui nécessitent de réaliser une large ouverture du corps du patient pour accéder aux tissus d'intérêt, les interventions endovasculaires ne demandent que de fines incisions pour pouvoir insérer les outils dans la structure vasculaire. Elles présentent plusieurs avantages, notamment une augmentation du taux de succès à court terme ainsi qu'une diminution de la morbidité peropératoire et de la durée d'hospitalisation. Malgré la généralisation de ces interventions, elles restent délicates et nécessitent d'être sécurisées et fiabilisées. L'accès aux tissus pathologiques est rendu difficile par la nature de l'intervention. La manipulation et le contrôle des instruments exigent une précision importante pour favoriser la réussite du traitement. Par ailleurs, le suivi des gestes opératoires ne peut être réalisé que par l'intermédiaire d'une imagerie peropératoire.

Dans le domaine des interventions endovasculaires on utilise des images bidimensionnelles acquises par fluoroscopie, permettant de guider l'insertion de dispositifs médicaux, tels des cathéters, dans l'artère fémorale et dans d'autres ramifications vasculaires.

La fluoroscopie désigne une technique d'imagerie médicale qui permet de visualiser des structures anatomiques en mouvement et en temps réel. Les artères étant des tissus mous et donc non visibles aux rayons X, on peut administrer au patient un produit de contraste radio-opaque afin de faire ressortir la structure vasculaire en indiquant le cheminement des artères.

Les images bidimensionnelles sont acquises et utilisées pendant une phase opératoire, donc une phase d'intervention.

Afin d'améliorer l'assistance apportée durant la phase opératoire, il a été proposé d'utiliser également des données d'image tridimensionnelles (ou images 3D), acquises pendant une phase préopératoire ou phase de planification, obtenues par des techniques d'acquisition comme par exemple la tomographie également appelée CT pour « computed tomography », l'imagerie par résonance magnétique (IRM). En effet, bien souvent, l'information bidimensionnelle concernant les organes n'est pas suffisante et les opérations requièrent une connaissance tridimensionnelle. Ces images 3D sont acquises avant l'opération pour le diagnostic de la maladie ou pour observer la forme de l'anévrisme et préparer l'intervention, et sont donc aisément disponibles lors de l'opération.

L'exploitation des informations tridimensionnelles générées en phase préopératoire lors de la navigation peropératoire nécessite une mise en correspondance de ces informations avec l'imagerie bidimensionnelle (2D) acquise pendant la phase d'intervention. La mise en correspondance est effectuée grâce à un processus de recalage qui permet d'exprimer les différentes données dans un même référentiel spatial.

L'objectif est de visualiser, en même temps et sur une même image, différents types d'informations telles que des images de différentes modalités ou des modèles anatomiques préalablement extraits. Cela permet de fournir au praticien des compléments d'information ainsi qu'une meilleure compréhension du champ opératoire, lui permettant d'améliorer la précision de l'intervention et de sécuriser le geste opératoire.

Il existe des systèmes de chirurgie guidée par l'image destinés à des salles d'opération dites hybrides, équipées d'un dispositif de support d'un dispositif d'acquisition d'image apte à tourner autour du patient pour l'acquisition d'images, par exemple par rayons X. Un tel dispositif de support connu sous le nom C-arm rotationnel motorisé, est utilisé en angiographie rotationnelle. L'utilisation d'un tel dispositif permet d'acquérir des données d'images tridimensionnelles pendant l'intervention et de faciliter la fusion entre données d'images acquises préalablement et données d'images acquises pendant l'intervention, par un recalage 3D/3D. La mise à jour du recalage est ensuite entièrement automatisée, et l'opérateur peut aisément changer les angles du C-arm ou déplacer la table du patient sans remettre en cause la fusion d'image. Au final, la quantité de produit de contraste à injecter est moindre et la durée de radiation est réduite.

Cependant, l'utilisation d'un C-arm rotationnel motorisé n'est pas pratique dans une salle d'opération, et peut gêner les mouvements du praticien. De plus, un tel équipement est cher et de nombreux hôpitaux ne peuvent pas en être équipés.

Dans la plupart des cas, les salles d'opération sont équipées d'un C-arm conventionnel, mobile et non motorisé, qui est un équipement plus léger et moins coûteux. Les objectifs d'assistance sont les mêmes qu'avec les C-arm motorisés, mais la fusion d'image n'est plus entièrement automatisée. Dans ce cas, le recalage est effectué entre l'image 3D acquise dans la phase préopératoire et les images 2D acquises pendant l'opération, et une mise à jour du recalage est nécessaire à chaque mouvement du patient, de la table ou du C-arm.

De plus, les images 2D acquises pendant la phase opératoire, appelées également images peropératoires, contiennent divers contenus qui rendent le recalage difficile. En effet, ces images contiennent des structures anatomiques de différents types : des structures osseuses constamment visibles, des structures vasculaires, totalement ou partiellement révélées grâce aux produits de contraste injectés, ainsi que des outils endovasculaires, par exemple cathéters, guides flexibles ou rigides, ou dispositifs de largage des endoprothèses. Par conséquent, il est généralement difficile de déterminer la transformation géométrique entre les données d'image préopératoires et les données d'image peropératoires.

De plus, les structures anatomiques subissent des déformations entre la phase préopératoire d'acquisition de données d'image 3D et la phase d'intervention, ces déformations pouvant être dues d'une part à des évolutions physiologiques, mais surtout à l'introduction d'outils endovasculaires dans les structures vasculaires du patient. En particulier, la structure vasculaire est déformée par l'introduction d'un guide rigide, qui a pour but de faciliter l'insertion par la suite d'un système de largage de l'endoprothèse. Le document WO03/088143 décrit un procédé d'assistance et de guidage de navigation d'un outil selon le préambule de la revendication 1.

L'invention a pour objet de remédier aux inconvénients des méthodes connues, pour améliorer les interventions endovasculaires guidées par l'image en permettant une meilleure prise en compte des déformations des structures vasculaires du patient en phase d'intervention.

A cet effet, l'invention décrit un système d'aide au guidage comme décrit dans le jeu de revendications.

Avantageusement, le procédé décrit ci-après comprend une combinaison d'une transformation rigide et d'une transformation élastique, permettant de générer un deuxième modèle tridimensionnel des structures vasculaires ciblées qui est déformé en cohérence avec les déformations de ces structures pendant la phase d'intervention. Ainsi, le deuxième modèle tridimensionnel est affiché en superposition des images bidimensionnelles acquises dans un contexte mis à jour par rapport à l'anatomie du patient, ce qui permet d'améliorer l'assistance apportée lors d'une intervention endovasculaire minimalement invasive.

Le procédé peut également présenter une ou plusieurs des caractéristiques ci-dessous, prises indépendamment ou en combinaison.

L'étape d'estimation d'une transformation élastique comporte une modélisation des structures vasculaires du patient à partir de l'image tridimensionnelle préopératoire acquise.

L'étape d'estimation d'une transformation élastique comporte une construction d'un modèle biomécanique d'interaction entre les structures vasculaires et l'outil endovasculaire.

Le procédé comporte une simulation de l'interaction entre l'outil endovasculaire et la structure vasculaire ciblée réalisée par une méthode d'analyse par éléments finis.

L'estimation de la transformation élastique comporte une étape supplémentaire de correction utilisant lesdites images bidimensionnelle peropératoires.

L'étape de correction comporte une projection de positions simulées de l'outil endovasculaire sur au moins une image bidimensionnelle peropératoire prise après introduction effective de l'outil endovasculaire, et une quantification de différence entre lesdites positions simulées et des positions effectives de l'outil endovasculaire.

Le procédé comprend en outre une étape de recalage entre lesdites positions simulées et lesdites positions effectives.

Le premier modèle anatomique tridimensionnel spécifique au patient est représenté par une structure parmi un volume, un maillage de points, un ensemble de contours et un ensemble de marqueurs anatomiques.

L'estimation d'une transformation rigide est effectuée par recalage automatique, semi-automatique ou manuel entre l'image tridimensionnelle préopératoire et au moins une image bidimensionnelle peropératoire.

L'invention concerne un système d'aide au guidage d'un outil endovasculaire dans des structures vasculaires, caractérisé en ce qu'il comprend un dispositif d'imagerie apte à acquérir des images bi-dimensionnelle de portions du corps d'un patient, un dispositif programmable et une unité de visualisation.

Le système est adapté à :
- lors d'une phase de planification préalable,
   - obtenir et mémoriser une image tridimensionnelle préopératoire comprenant une structure vasculaire ciblée d'un patient,
   - déterminer un premier modèle anatomique tridimensionnel spécifique au patient à partir de l'image tridimensionnelle acquise, ce premier modèle anatomique tridimensionnel étant situé dans un même référentiel spatial que l'image tridimensionnelle, -lors d'une phase d'intervention,
   - acquérir une ou plusieurs images bidimensionnelles peropératoires comprenant la structure vasculaire ciblée du patient, opacifiée ou non,
   - estimer une transformation rigide entre l'image tridimensionnelle préopératoire et les images bidimensionnelles peropératoires,
   - estimer une transformation élastique entre ladite image tridimensionnelle et les images bidimensionnelles en fonction de la transformation rigide et d'une simulation de déformations vasculaires induites par une introduction de l'outil dans la structure vasculaire ciblée,
   - appliquer une combinaison de la transformation rigide et de la transformation élastique au premier modèle anatomique tridimensionnel spécifique au patient pour obtenir un deuxième modèle tridimensionnel spécifique au patient,
   - afficher sur l'unité de visualisation ledit deuxième modèle tridimensionnel spécifique au patient en superposition sur les images bidimensionnelles acquises.

Selon un deuxième aspect, l'invention concerne un programme d'ordinateur comportant des instructions pour mettre en œuvre les étapes d'un procédé d'aide au guidage d'un outil endovasculaire dans des structures vasculaires tel que brièvement décrit ci-dessus, lors de l'exécution du programme par un processeur d'un dispositif programmable.

D'autres caractéristiques et avantages de l'invention ressortiront de la description qui en est donnée ci-dessous, à titre indicatif et nullement limitatif, en référence aux figures annexées, parmi lesquelles :
- la figure 1 représente schématiquement un système d'intervention endovasculaire guidée par l'image;
- la figure 2 est un synoptique des principaux blocs d'un dispositif programmable apte à mettre en œuvre le procédé de l'invention ;
- la figure 3 est un organigramme d'un procédé d'aide au guidage d'un outil endovasculaire;
- la figure 4 est un organigramme des étapes de la détermination d'une transformation élastique selon un mode de réalisation de l'invention ;
- la figure 5 est un exemple d'images de structures vasculaires après recalage ;
- la figure 6 est un exemple de fusion d'image 2D peropératoire et d'un modèle anatomique avant et après application de la transformation élastique ;
- la figure 7 est un exemple d'affichage enrichi comportant un modèle anatomique après application de la transformation élastique.

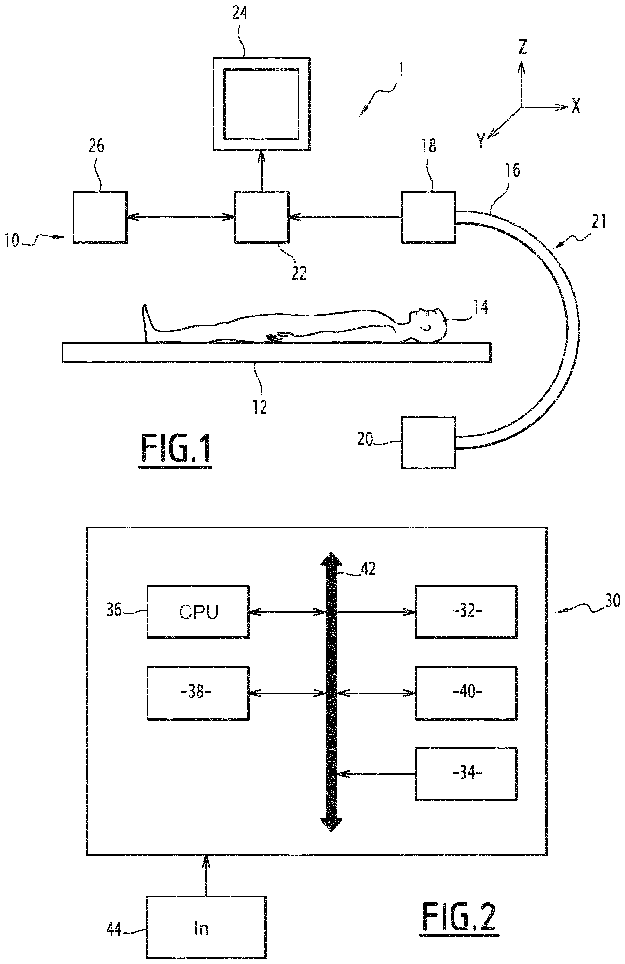

La figure 1 illustre schématiquement une salle d'opération 1, équipée d'un système 10 d'intervention endovasculaire guidée par l'image.

La salle d'opération 1 est équipée d'une table d'opération 12, sur laquelle est représenté un patient 14 à traiter par une intervention endovasculaire.

Le système d'intervention 10 comporte un dispositif 21 d'imagerie à rayon X, lui-même composé d'un dispositif de support 16 en forme d'arceau, d'une source 18 de rayon X et d'une unité 20 de réception et de détection de rayons X, positionnée en regard de la source 18. Ce dispositif d'imagerie est apte à capter des images des éléments positionnés entre la source 18 de rayons X et l'unité 20 de réception et de détection, et est également apte à tourner autour de deux axes, l'axe X et l'axe Y selon le besoin de l'opérateur.

Ainsi, le dispositif d'imagerie 21 illustré est apte à capter des images bidimensionnelles radiographiques de diverses portions du corps du patient, comprenant des structures vasculaires ciblées.

Le système d'intervention 10 comporte également un dispositif programmable 22, comportant un ou plusieurs processeurs, associé à une unité de visualisation 24 composée d'un ou de plusieurs écrans et d'une interface homme-machine 26.

L'interface homme-machine 26 comprend des moyens de pointage et de sélection d'éléments, par exemple un ensemble clavier-souris, un pavé tactile, une interface gestuelle 3D sans contact ou une combinaison de ces dispositifs.

Dans un mode de réalisation, l'interface homme-machine 26 est intégrée avec l'unité de visualisation 24 sous la forme d'un écran tactile.

Le dispositif programmable 22 est adapté à recevoir les images bidimensionnelles radiographiques acquises par le dispositif d'imagerie à rayons X et à les traiter selon un procédé d'aide au guidage d'un outil endovasculaire dans des structures vasculaires selon l'invention.

Les images bidimensionnelles acquises pendant la phase d'intervention sont affichées sur l'unité de visualisation 24, ainsi qu'un modèle tridimensionnel spécifique au patient, permettant un guidage plus précis des outils endovasculaires dans un contexte mis à jour par rapport à l'anatomie du patient.

Les outils endovasculaires sont sélectionnés parmi un cathéter, un dispositif endovasculaire de type stent, un guide souple ou rigide, un cathéter, une endoprothèse ou un ballon.

La figure 2 illustre les principaux blocs d'un dispositif programmable 30 apte à mettre en œuvre le procédé d'aide au guidage d'un outil endovasculaire dans des structures vasculaires selon un mode de réalisation de l'invention.

Un dispositif programmable 30 apte à mettre en œuvre l'invention, comprend un écran 32, analogue à l'unité de visualisation 24, une unité 34 de saisie des commandes d'un opérateur, par exemple un clavier, une souris, un pavé tactile ou une interface sans contact, une unité centrale de traitement 36, ou CPU, apte à exécuter des instructions de programme informatique lorsque le dispositif 30 est mis sous tension. Le dispositif 30 comporte optionnellement un contrôleur 40, permettant d'envoyer des commandes et de sélectionner des éléments à distance.

Le dispositif 30 comporte également une unité de stockage d'informations 38, par exemple des registres, aptes à stocker des instructions de code exécutable permettant la mise en œuvre de programmes comportant des instructions de code aptes à mettre en œuvre le procédé selon l'invention. Les divers blocs fonctionnels du dispositif 30 décrits ci-dessus sont connectés via un bus de communication 42.

Le dispositif 30 est apte à recevoir des données d'image d'une source 44.

Le procédé est adapté à être mis en œuvre par un dispositif programmable tel un ordinateur intégré dans une salle d'intervention standard, ce qui permet de limiter les coûts d'équipement.

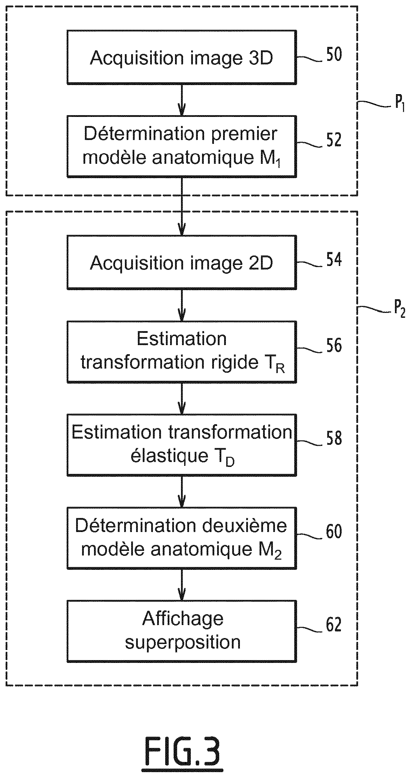

La figure 3 représente les principales étapes mises en œuvre dans un procédé d'aide au guidage d'un outil endovasculaire dans des structures vasculaires mises en œuvre par un processeur 36 d'un dispositif programmable 30.

Le procédé comporte deux phases, une phase de planification préopératoire P₁, qui est effectuée préalablement à l'intervention et dont les résultats sont mémorisés, et une phase d'intervention P₂, effectuée pendant une intervention d'un praticien sur un patient.

Dans le mode de réalisation illustré à la figure 3, la phase de planification P₁ comporte la mise en œuvre de deux étapes.

Une première étape 50 consiste à acquérir et à mémoriser une image 3D préopératoire d'une partie du corps du patient comprenant une structure vasculaire à traiter du patient.

Par exemple, pour un anévrisme aortique abdominal, l'image 3D acquise contient l'aorte abdominale.

Par exemple, l'image 3D, également appelée image volumique, est obtenue par la technique de tomographie connue sous le nom de CT.

En variante, d'autres techniques connues comme l'angioscanner ou l'IRM sont utilisées.

Ces techniques d'acquisition d'images 3D sont connues dans le domaine de l'imagerie médicale et ne sont pas décrites plus en détail ici.

L'image 3D préopératoire est mémorisée dans un format approprié dans une mémoire du dispositif programmable mettant en œuvre le procédé de l'invention.

Avantageusement, l'image 3D obtenue est représentative de l'anatomie du patient à traiter, et permet de prendre des mesures et de dimensionner les outils endovasculaires à utiliser lors de l'intervention.

L'étape 50 d'acquisition d'image 3D d'une structure vasculaire du patient est suivie, dans la phase de planification P₁, d'une étape 52 de détermination d'un premier modèle anatomique tridimensionnel virtuel du patient (modèle M₁) comprenant un modèle de la structure vasculaire ciblée.

Ce premier modèle anatomique tridimensionnel virtuel est obtenu par traitement d'image appliqué à l'image 3D, par application d'algorithmes automatiques ou semi-automatiques.

Par exemple, un algorithme de segmentation semi-automatique de type coupe de graphe peut être utilisé. Cet algorithme, peu couteux en termes d'interaction avec l'utilisateur, offre des résultats rapides et précis et permet d'obtenir une segmentation précise de l'aorte et des artères iliaques internes et rénales.

Selon l'algorithme utilisé, le premier modèle anatomique tridimensionnel virtuel est représenté soit sous la forme d'un volume (image 3D), un maillage de points (ou « mesh » en anglais), un ensemble de contours, des marqueurs anatomique ou une combinaison de ces éléments. La représentation du premier modèle anatomique tridimensionnel virtuel est mémorisée pour utilisation dans la phase d'intervention P₂.

Par exemple, un tel premier modèle anatomique tridimensionnel virtuel M₁ peut comprendre les volumes segmentés de l'aorte, des artères iliaques internes et des artères rénales, de la colonne vertébrale ainsi que des plaques calcifiées.

La phase de planification préopératoire P1 est suivie de la phase d'intervention P2.

Lors d'une étape 54 d'acquisition d'images en phase d'intervention, plusieurs images 2D peropératoires comprenant une région anatomique d'intérêt du patient sont obtenues. La région anatomique d'intérêt comprend la structure vasculaire ciblée, opacifiée ou non par des produits de contraste.

Selon un mode de réalisation, l'acquisition d'images se fait par un dispositif d'imagerie 21 fluoroscopique à rayons X comprenant un dispositif de support de type C-arm tel qu'illustré à la figure 1. Ces images sont obtenues en temps-réel et forment un flux vidéo. Les structures vasculaires peuvent être momentanément rendues visibles grâce à un produit de contraste injecté.

En variante, des images 2D peropératoires sont obtenues par un dispositif d'acquisition d'images par ultrasons.

La pluralité d'images 2D peropératoires acquises à l'étape d'acquisition 54 comprend au moins une image 2D de la région anatomique d'intérêt.

L'étape 54 d'acquisition d'images 2D peropératoires est suivie d'une étape 56 d'estimation d'une transformation rigide T_{R} permettant d'effectuer un recalage spatial entre l'image 3D préopératoire acquise lors de l'étape 50 et les images 2D peropératoires acquises lors de l'étape 54. La transformation rigide estimée permet de mettre en correspondance les structures d'intérêt de chaque image 2D peropératoire avec celles de l'image 3D préopératoire. Les structures d'intérêt comprennent par exemple des structures osseuses constamment visibles.

En d'autres termes, il s'agit d'estimer une transformation rigide T_{R} qui met en correspondance le référentiel spatial de l'image 3D et du premier modèle anatomique tridimensionnel virtuel avec le référentiel spatial des images acquises en phase opératoire.

Selon un premier mode de réalisation, l'étape 56 met en œuvre l'affichage simultané des images 3D et des images 2D acquises, et un recalage manuel, effectué par inspection visuelle, de plusieurs points d'intérêt des images 3D et des images 2D inspectées.

Selon une alternative, un algorithme de recalage automatique est mis en œuvre. Il est à noter que de nombreux algorithmes de recalage 3D/2D sont connus dans le domaine de l'imagerie médicale.

Par exemple, un recalage automatique de type iconique peut être mis en œuvre, en utilisant une mesure de similarité basée sur la différence de gradients entre l'image 3D préopératoire et une ou plusieurs images 2D peropératoires, couplé à une stratégie d'optimisation de type descente de gradient ou optimiseur de Powell.

Selon une autre variante, un recalage semi-automatique est mis en œuvre, dans lequel la détermination d'une transformation initiale est effectuée manuellement. Dans ce type de méthode, l'initialisation manuelle permet de faire correspondre grossièrement les deux images à recaler, et le recalage automatique est ensuite lancé par la suite pour affiner le résultat.

A l'issue de l'étape 56, les valeurs des paramètres définissant la transformation rigide estimée sont mémorisées.

Avantageusement, l'étape 56 de détermination d'une transformation rigide peut être effectuée à divers stades de l'intervention, par exemple avant l'introduction d'outils endovasculaires dans la structure vasculaire du patient, ou après cette introduction.

De même, l'étape 56 de détermination d'une transformation rigide peut être effectuée à partir d'une ou plusieurs images 2D peropératoires, acquises avec ou sans injection d'un produit de contraste, montrant la structure osseuse ou la structure vasculaire, avec la présence d'outils ou non, ainsi qu'en utilisant un angle unique d'incidence du C-arm ou plusieurs.

A ce stade, le recalage n'est pas suffisamment précis pour permettre de faire correspondre parfaitement l'image 3D préopératoire avec les images 2D peropératoires. L'étape 56 de détermination d'une transformation rigide est donc suivie d'une étape 58 d'estimation d'une transformation élastique d'ajustement T_{D}, permettant d'améliorer le recalage entre l'image 3D préopératoire et les images 2D peropératoires.

L'estimation de la transformation élastique est basée sur une déformation des structures anatomiques due à l'introduction d'un outil endovasculaire dans les structures vasculaires du patient.

Il est considéré que l'outil introduit, par exemple un guide ou un cathéter, est la cause principale des déformations, et que les déformations introduites sont modélisables par un modèle biomécanique spécifique.

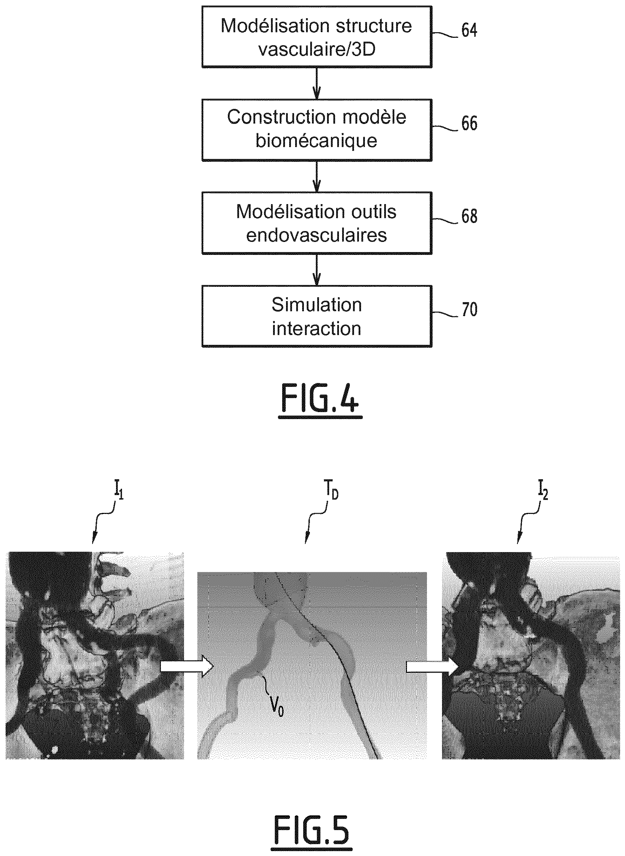

Un mode de réalisation de la détermination ou estimation d'une transformation élastique sera décrit plus en détail ci-après en référence à la figure 4.

L'étape de détermination d'une transformation élastique est suivie d'une étape 60 de combinaison de la transformation rigide T_{R} et des paramètres de déformation de la transformation élastique T_{D} pour obtenir une transformation finale T_{F}.

La transformation géométrique finale permet de mettre en correspondance chaque point des images 2D peropératoires avec un point ou voxel de l'espace des images 3D préopératoires.

La transformation géométrique finale est appliquée au premier modèle anatomique tridimensionnel virtuel M₁ pour obtenir un deuxième modèle anatomique tridimensionnel virtuel déformé M₂.

Ce deuxième modèle anatomique tridimensionnel M₂ est plus précis car il tient compte des déformations courantes des structures vasculaires pendant la phase d'intervention.

Par exemple, la transformation finale T_{F} est appliquée à tous les points de maillage définissant le modèle M₁ afin d'obtenir des points de maillage définissant le modèle M₂, et ces points de maillage définissant le modèle M₂ sont mémorisés.

Finalement, lors d'une étape d'affichage 62, le deuxième modèle anatomique tridimensionnel obtenu est affiché en superposition avec les images 2D peropératoires.

L'aide à la navigation endovasculaire et au guidage de l'introduction d'un outil endovasculaire dans la structure vasculaire du patient est ainsi améliorée, car le modèle anatomique spécifique au patient est plus précis et mieux adapté au contexte peropératoire.

Avantageusement, l'assistance fournie par l'affichage de la fusion d'images permet donc de sécuriser, fiabiliser et guider le geste interventionnel par l'apport d'informations pertinentes extraites en phase préopératoire et rapportées au sein de la salle d'opération. Elle doit permettre à long terme de réduire les injections de produit de contraste et les émissions de rayons X.

La figure 4 illustre un mode de réalisation de l'étape de détermination d'une transformation élastique.

La détermination d'une transformation élastique comporte une première étape 64 de modélisation de la structure vasculaire ciblée du patient réalisée à partir des données préopératoires, en particulier à partir des images 3D mémorisées La modélisation consiste à créer une représentation géométrique de la structure vasculaire exploitable pour estimer les déformations vasculaires.

Dans un mode de réalisation, la structure vasculaire est modélisée par des courbes B-splines correspondant aux contours du premier modèle anatomique. Une interpolation est réalisée entre les courbes B-splines afin de créer un modèle surfacique représentant la paroi vasculaire.

Ensuite, une étape 66 de construction d'un modèle biomécanique spécifique au patient est mise en œuvre. Elle consiste à définir le comportement mécanique de la structure vasculaire en attribuant des propriétés mécaniques à la représentation géométrique obtenue à l'étape 64. Le modèle biomécanique doit prendre en compte les spécificités du patient, à savoir l'état local de la paroi vasculaire (sain, calcifié) et la relation entre la structure vasculaire et son environnement direct (interactions avec la structure osseuse et les tissus mous environnants).

Dans un mode de réalisation, une simulation des interactions entre les outils et la structure vasculaire, tel que décrite en référence à l'étape 70 ci-après, est réalisée pour un ensemble de patients dit d'apprentissage. L'erreur de simulation est quantifiée en mesurant l'écart entre la forme des outils simulés et celle des outils réels observables sur une ou plusieurs images peropératoires. Les paramètres du modèle biomécanique définissant la relation entre la structure vasculaire et son environnement direct sont progressivement ajustés dans le but de minimiser l'erreur de simulation.

Des expressions reliant des données préopératoires issues des images 3D mémorisées avec la valeur des paramètres du modèle biomécanique sont définies à partir des valeurs optimales obtenues au terme de l'ajustement progressif des paramètres du modèle biomécanique pour chaque patient. Ces expressions définissent un modèle biomécanique adaptatif entièrement défini à partir de données préopératoires issues des images 3D mémorisées et de connaissances anatomiques et mécaniques.

Dans un mode de réalisation, les valeurs utilisées pour la rigidité des tissus sont issues des données moyennes issues de caractérisations reportées dans la littérature, alors que l'état de calcification des artères est pris en compte de façon spécifique pour chaque patient en attribuant une rigidité différente aux zones calcifiées et aux zones saines distinguables sur l'imagerie préopératoire. Pour les niveaux de déformation atteints lors des simulations, l'anisotropie de la paroi peut être négligée.

L'étape suivante 68 de modélisation des outils endovasculaires consiste à créer une représentation géométrique des outils endovasculaires selon leur forme réelle et à définir une loi de comportement selon leurs propriétés mécaniques.

Par exemple, un guide rigide utilisé dans les procédures mini-invasives peut être modélisé par des éléments 1D de type poutre de section circulaire. Pour les dispositifs de largage, une structure tubulaire de section homogène auquel un matériau de rigidité équivalente est affecté pourra être utilisée pour la modélisation.

Dans un mode de réalisation, les propriétés mécaniques des outils endovasculaires sont caractérisées au moyen d'essais mécaniques permettant d'établir une relation entre la force appliquée à l'outil et la déformation de l'outil. Par exemple, un test de flexion 3 points peut être réalisé pour quantifier la rigidité en flexion des outils endovasculaires. Les propriétés ainsi caractérisées sont utilisées dans les simulations pour représenter le comportement mécanique réel des outils.

L'étape de modélisation des outils endovasculaires 68 est suivie d'une étape 70 de simulation de l'interaction des outils endovasculaires introduits ou à introduire dans la structure vasculaire avec la paroi de la structure vasculaire du patient.

Dans un mode de réalisation, la simulation est réalisée à l'aide d'une analyse par éléments finis. La représentation géométrique du modèle biomécanique spécifique au patient et du modèle des outils endovasculaires est discrétisée en un ensemble d'éléments possédant une forme prédéterminée (éléments coques triangulaires, éléments poutres, éléments hexaèdres...).

Le comportement mécanique de chaque élément est défini selon le comportement mécanique précédemment attribué aux modèles de la structure vasculaire et des outils endovasculaires. Les interactions entre les deux modèles sont gérées par une méthode de gestion de contact, par exemple par une méthode de pénalisation.

Dans un mode de réalisation, les conditions aux limites sont définies au regard de considérations anatomiques et mécaniques issues de la littérature et des connaissances des experts chirurgiens. Par exemple, les extrémités proximales et distales de la structure vasculaire sont fixées et le lien entre la structure vasculaire et les tissus environnants est modélisé par une rigidité supplémentaire.

Dans un mode de réalisation, une précontrainte vasculaire est appliquée avant de simuler les interactions outils/tissus. Son rôle est de prendre en compte l'état de repos de la structure vasculaire. Avant de simuler les interactions outils/tissus, l'état de pré-tension de la paroi vasculaire dû à la présence de la pression sanguine est pris en compte. Pour cela une géométrie correspondant à l'état de repos de la structure vasculaire (pression sanguine nulle), dite géométrie « zéro-pression » est déterminée, puis la pression artérielle est appliquée dans cette géométrie "zéro-pression" afin de mettre en tension la paroi vasculaire.

La géométrie de la paroi vasculaire est déterminée en particulier par des paramètres de diamètre, longueur, angulation, épaisseur.

La géométrie de référence observée sur l'image pré-opératoire est choisie comme première estimation de l'état zéro-pression, puis est corrigée de façon itérative à chaque pas de l'algorithme. Pour cela, chaque itération consiste à appliquer la pression interne dans la géométrie zéro-pression puis à comparer la géométrie finale ainsi obtenue avec la géométrie de référence. La géométrie zéro-pression est alors corrigée par application de l'opposé des écarts de position observés.

La simulation de l'interaction des outils endovasculaires avec la paroi de la structure vasculaire du patient est réalisée en initialisant les outils à l'intérieur de la structure vasculaire par des contraintes imposées, par exemple de type déplacements imposés, puis en observant les déformations vasculaires provoquées par les outils lorsque les contraintes imposées sont progressivement annulées.

L'initialisation consiste à contraindre l'outil à l'intérieur de la lumière vasculaire, par exemple sur un chemin minimisant son énergie de flexion. Une fois à l'intérieur de la lumière vasculaire, le contact entre la face interne de la paroi vasculaire et l'outil est activé. Enfin les contraintes nécessaires à l'initialisation sont progressivement relâchées. Un équilibre mécanique va alors s'établir entre l'outil et la structure vasculaire générant les déformations vasculaires que l'on souhaite calculer.

Dans un mode de réalisation de la simulation alternatif, les outils sont insérés progressivement à l'intérieur de la structure vasculaire jusqu'à leur insertion complète. Les déformations vasculaires peuvent être calculées à chaque sous-étape de l'insertion progressive.

Les étapes décrites précédemment définissent un mode de réalisation pour définir la transformation élastique.

Dans un mode de réalisation alternatif, la détermination d'une transformation élastique comporte une étape supplémentaire de correction. Cette étape consiste à projeter des positions simulées de l'outil introduit sur une ou plusieurs images 2D peropératoires, à quantifier la différence entre les positions simulées et les positions effectives de l'outil réel, et à utiliser un modèle de recalage 2D/2D pour déterminer une correction de la transformation élastique. Un algorithme de recalage 2D/2D connu peut être utilisé, tel qu'un recalage utilisant une approche géométrique

La figure 5 illustre une structure vasculaire V₀, le résultat I₁ de la transformation rigide T_{R} appliquée à une image 3D préopératoire représentée en coupe, et le résultat I₂ de la transformation finale T_{F}, issues de la combinaison de la transformation rigide et de la transformation élastique, appliquée à la même image 3D préopératoire.

Au centre, on illustre le résultat de la transformation élastique.

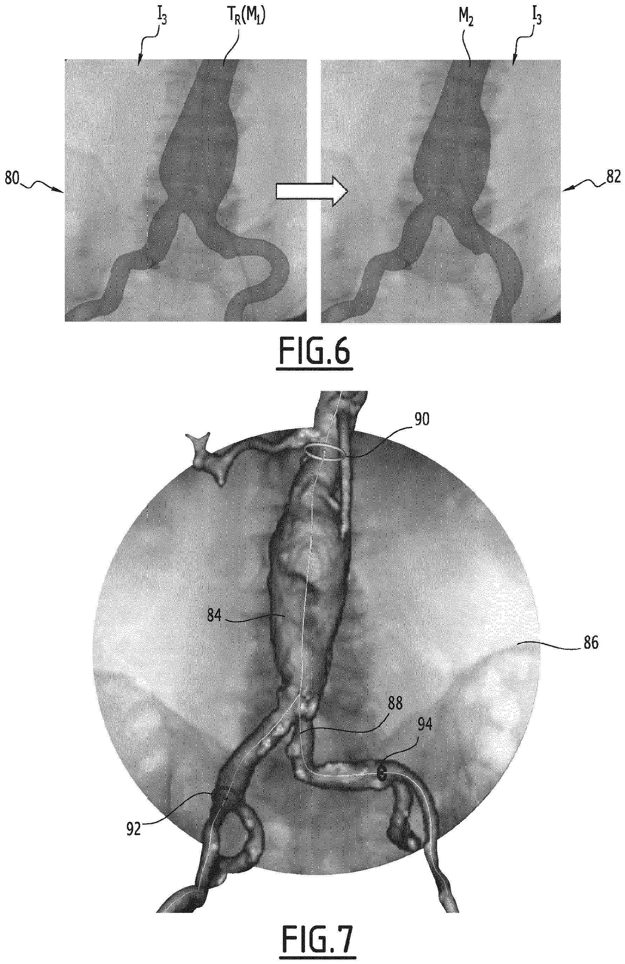

La figure 6 illustre schématiquement une fusion d'image 2D peropératoire I₃ et d'un modèle anatomique tridimensionnel pour faciliter le guidage et la navigation de l'introduction d'outil endovasculaire.

Dans l'illustration 80, la transformation rigide T_{R} du premier modèle anatomique tridimensionnel M₁ est projetée sur l'image peropératoire I₃.

Dans l'illustration 82 le deuxième modèle anatomique tridimensionnel M₂ est projeté sur l'image peropératoire I₃.

De préférence, une fonction d'opacité est appliquée à l'image peropératoire 2D, permettant d'améliorer la visualisation simultanée des outils endovasculaires et du modèle anatomique tridimensionnel projeté.

De plus, l'invention permet d'améliorer le guidage et la navigation dans des structures vasculaires par affichage d'informations supplémentaires obtenues à partir du deuxième modèle anatomique tridimensionnel qui est représentatif des déformations des structures vasculaires au moment de l'intervention.

La figure 7 illustre un tel modèle anatomique tridimensionnel après déformation 84 affiché en superposition d'une image 2D peropératoire 86. L'affichage est ainsi enrichi d'informations supplémentaires en réalité augmentée.

L'affichage contient également une ligne centrale aortique 88 obtenue par calcul, ainsi que des marqueurs anatomiques 90, 92, 94 superposés à des points d'intérêt anatomique, à savoir l'ostium 90, et les points de départ de l'artère iliaque droite 92 et gauche 94.

Tous les marqueurs anatomiques ont été préalablement extraits du premier modèle anatomique tridimensionnel, et sont des indications visuelles permettant d'enrichir l'ensemble des informations fournies au praticien.

Avantageusement, une utilisation du modèle anatomique tridimensionnel déformé fourni par l'invention est le suivi de l'outil endovasculaire inséré, permettant de fournir une localisation spatiale de l'outil en temps réel. Ce suivi se base sur des marqueurs radio opaques, un système infrarouge ou un système électromagnétique.

Selon une autre variante, le suivi d'outils est effectué manuellement par sélection de l'outil endovasculaire suivi dans l'image affichée.

L'invention trouve des applications dans diverses procédures nécessitant des interventions endovasculaires, en particulier le traitement d'anévrisme, de traumastismes vasculaires ou de l'artériosclérose.

## Revendications

1. Système d'aide au guidage d'un outil endovasculaire dans des structures vasculaires, comprenant un dispositif d'imagerie apte à acquérir des images bi-dimensionnelle de portions du corps d'un patient, un dispositif programmable et une unité de visualisation, ledit système étant adapté à ;
- lors d'une phase de planification (P₁) préalable,
• obtenir et mémoriser (50) une image tridimensionnelle préopératoire comprenant une structure vasculaire ciblée d'un patient,
• déterminer (52) un premier modèle anatomique tridimensionnel (M₁) spécifique au patient à partir de l'image tridimensionnelle acquise, ce premier modèle anatomique tridimensionnel (M₁) étant situé dans un même référentiel spatial que l'image tridimensionnelle,
- lors d'une phase d'intervention (P₂),
• acquérir (54) une ou plusieurs images bidimensionnelles peropératoires comprenant la structure vasculaire ciblée du patient, opacifiée ou non,
• estimer (56) une transformation rigide entre l'image tridimensionnelle préopératoire et les images bidimensionnelles peropératoires, ledit système étant **caractérisé en ce qu'**il est par ailleurs adapté à:
• estimer (58) une transformation élastique entre ladite image tridimensionnelle et les images bidimensionnelles en fonction de la transformation rigide et d'une simulation de déformations vasculaires induites par une introduction de l'outil dans la structure vasculaire ciblée, ladite simulation étant obtenue en fonction d'un état local de la structure vasculaire ciblée, d'une relation entre la structure vasculaire ciblée et son environnement direct, et de propriétés mécaniques de l'outil endovasculaire,
• appliquer (60) une combinaison de la transformation rigide et de la transformation élastique au premier modèle anatomique tridimensionnel (M₁) spécifique au patient pour obtenir un deuxième modèle tridimensionnel (M₂) spécifique au patient,
• afficher (62) sur l'unité de visualisation ledit deuxième modèle tridimensionnel spécifique au patient en superposition sur les images bidimensionnelles acquises.

2. Système selon la revendication 1, ledit système étant adapté pour modéliser (64) des structures vasculaires du patient à partir de l'image tridimensionnelle préopératoire acquise lors de ladite estimation (58) d'une transformation élastique.

3. Système selon l'une des revendications 1 ou 2, ledit système étant adapté pour construire (66) un modèle biomécanique d'interaction entre les structures vasculaires et l'outil endovasculaire lors de ladite estimation (58) d'une transformation élastique.

4. Système selon la revendication 3, ledit système étant adapté pour réaliser une simulation (68, 70) de l'interaction entre l'outil endovasculaire et la structure vasculaire ciblée par une méthode d'analyse par éléments finis.

5. Système selon l'une quelconque des revendications 1 à 4, ledit système étant en outre adapté pour mettre en œuvre une correction utilisant lesdites images bidimensionnelle peropératoires lors de ladite estimation (58) d'une transformation élastique.

6. Système selon la revendication 5, ledit système étant en outre adapté pour réaliser, lors de ladite correction, une projection de positions simulées de l'outil endovasculaire sur au moins une image bidimensionnelle peropératoire prise après introduction effective de l'outil endovasculaire, et une quantification de différence entre lesdites positions simulées et des positions effectives de l'outil endovasculaire.

7. Système selon la revendication 6, ledit système étant en outre adapté pour effectuer un recalage entre lesdites positions simulées et lesdites positions effectives.

8. Système selon l'une quelconque des revendications 1 à 7, dans lequel le premier modèle anatomique tridimensionnel spécifique au patient est représenté par une structure parmi un volume, un maillage de points, un ensemble de contours et un ensemble de marqueurs anatomiques.

9. Système selon l'une quelconque des revendications 1 à 8, dans lequel la détermination d'une transformation rigide est effectuée par recalage automatique, semi-automatique ou manuel entre l'image tridimensionnelle préopératoire et au moins une image bidimensionnelle peropératoire.

10. Produit programme d'ordinateur comportant des instructions pour mettre en œuvre les étapes suivantes d'un procédé d'aide au guidage d'un outil endovasculaire dans des structures vasculaires lors de l'exécution du programme par un processeur d'un dispositif programmable :
- lors d'une phase de planification (P₁) préalable,
• obtention (50) et mémorisation d'une image tridimensionnelle préopératoire comprenant une structure vasculaire ciblée d'un patient,
• détermination (52) d'un premier modèle anatomique tridimensionnel (M₁) spécifique au patient à partir de l'image tridimensionnelle acquise, ce premier modèle anatomique tridimensionnel étant situé dans un même référentiel spatial que l'image tridimensionnelle,
- lors d'une phase d'intervention (P₂),
• acquisition (54) d'une ou plusieurs images bidimensionnelles peropératoires comprenant la structure vasculaire ciblée du patient, opacifiée ou non,
• estimation (56) d'une transformation rigide entre l'image tridimensionnelle préopératoire et les images bidimensionnelles peropératoires,
• estimation (58) d'une transformation élastique entre ladite image tridimensionnelle et les images bidimensionnelles en fonction de la transformation rigide et d'une simulation de déformations vasculaires induites par une introduction de l'outil dans la structure vasculaire ciblée, ladite simulation étant obtenue en fonction d'un état local de la structure vasculaire ciblée, d'une relation entre la structure vasculaire ciblée et son environnement direct, et de propriétés mécaniques de l'outil endovasculaire,
• application (60) d'une transformation finale, combinaison de la transformation rigide et de la transformation élastique, au premier modèle anatomique tridimensionnel (M₁) spécifique au patient pour obtenir un deuxième modèle tridimensionnel (M₂) spécifique au patient,
• affichage (62) dudit deuxième modèle tridimensionnel spécifique au patient en superposition sur les images bidimensionnelles acquises.

## Patentansprüche

1. System zur Unterstützung der Führung eines endovaskulären Werkzeugs in vaskulären Strukturen, umfassend eine Bildgebungsvorrichtung, die geeignet ist, zweidimensionale Bilder von Abschnitten des Körpers eines Patienten aufzunehmen, eine programmierbare Vorrichtung und eine Anzeigeeinheit, wobei das System dazu eingerichtet ist:
- während einer vorherigen Planungsphase (P₁),
- ein dreidimensionales präoperatives Bild umfassend eine vaskuläre Zielstruktur eines Patienten aufzunehmen und zu speichern (50),
- ein erstes dreidimensionales anatomisches Modell (M₁), das für den Patienten spezifisch ist, aus dem aufgenommenen dreidimensionalen Bild zu bestimmen (52), wobei sich dieses erste dreidimensionale anatomische Modell (M₁) in einem gleichen räumlichen Bezugsrahmen wie das dreidimensionale Bild befindet,
- während einer Interventionsphase (P₂)
- ein oder mehrere präoperative zweidimensionale Bilder aufzunehmen (54), die die vaskuläre Zielstruktur des Patienten, abgedunkelt oder nicht, umfasst,
- eine starre Transformation zwischen dem präoperativen dreidimensionalen Bild und den präoperativen zweidimensionalen Bildern zu schätzen (56),
wobei das System **dadurch gekennzeichnet ist, dass** es überdies dazu eingerichtet ist:
- eine elastische Transformation zwischen dem dreidimensionalen Bild und den zweidimensionalen Bildern in Abhängigkeit von der starren Transformation und einer Simulation von vaskulären Deformationen, die durch eine Einführung des Werkzeugs in die vaskuläre Zielstruktur induziert werden, zu schätzen (58), wobei die Simulation in Abhängigkeit von einem lokalen Zustand der vaskulären Zielstruktur, einer Relation zwischen der vaskulären Zielstruktur und ihrer direkten Umgebung und von mechanischen Eigenschaften des endovaskulären Werkzeugs erhalten wird,
- eine Kombination der starren Transformation und der elastischen Transformation an dem ersten dreidimensionalen anatomischen Modell (M₁), das für den Patienten spezifisch ist, anzuwenden (60), um ein zweites dreidimensionales Modell (M₂), das für den Patienten spezifisch ist, zu erhalten,
- auf der Anzeigeeinheit das zweite dreidimensionale Modell, das für den Patienten spezifisch ist, in Überlagerung über die aufgenommenen zweidimensionalen Bilder anzuzeigen (62).

2. System nach Anspruch 1, wobei das System dazu eingerichtet ist, vaskuläre Strukturen des Patienten aus dem präoperativen dreidimensionalen Bild, das bei der Schätzung (58) einer elastischen Transformation aufgenommen wurde, zu modellieren (64).

3. System nach einem der Ansprüche 1 oder 2, wobei das System dazu eingerichtet ist, ein biomechanisches Interaktionsmodell zwischen den vaskulären Strukturen und dem endovaskulären Werkzeug bei der Schätzung (58) einer elastischen Transformation zu konstruieren (66).

4. System nach Anspruch 3, wobei das System dazu eingerichtet ist, eine Simulation (68, 70) der Interaktion zwischen dem endovaskulären Werkzeug und der vaskulären Zielstruktur durch eine Finite-Elemente-Analysemethode durchzuführen.

5. System nach einem der Ansprüche 1 bis 4, wobei das System ferner dazu eingerichtet ist, eine Korrektur einzusetzen, die die präoperativen zweidimensionalen Bilder bei der Schätzung (58) einer elastischen Transformation verwendet.

6. System nach Anspruch 5, wobei das System ferner dazu eingerichtet ist, bei der Korrektur eine Projektion von simulierten Positionen des endovaskulären Werkzeugs auf mindestens ein präoperatives zweidimensionales Bild, das nach der tatsächlichen Einführung des endovaskulären Werkzeugs aufgenommen wurde, und eine Quantifikation einer Differenz zwischen den simulierten Positionen und den tatsächlichen Positionen des endovaskulären Werkzeugs durchzuführen.

7. System nach Anspruch 6, wobei das System ferner dazu eingerichtet ist, eine Neueinstellung zwischen den simulierten Positionen und den effektiven Positionen vorzunehmen.

8. System nach einem der Ansprüche 1 bis 7, bei dem das erste dreidimensionale anatomische Modell, das für den Patienten spezifisch ist, durch eine Struktur wie Volumen, Netz von Punkten, Gesamtheit von Konturen und Gesamtheit von anatomischen Markern dargestellt wird.

9. System nach einem der Ansprüche 1 bis 8, bei dem die Bestimmung einer starren Transformation durch automatische, halb-automatische oder manuelle Neueinstellung zwischen dem präoperativen dreidimensionalen Bild und mindestens einem präoperativen zweidimensionalen Bild erfolgt.

10. Computerprogrammprodukt, umfassend Anweisungen für den Einsatz der folgenden Schritte eines Verfahrens zur Unterstützung der Führung eines endovaskulären Werkzeugs in vaskulären Strukturen bei der Ausführung eines Programms durch einen Prozessor einer programmierbaren Vorrichtung:
- während einer Planungsphase (P₁) vorab
- Aufnahme (50) und Speicherung eines dreidimensionalen präoperativen Bildes, umfassend eine vaskuläre Zielstruktur eines Patienten,
- Bestimmung (52) eines ersten dreidimensionalen anatomischen Modells (M₁), das für den Patienten spezifisch ist, aus dem aufgenommenen dreidimensionalen Bild, wobei sich dieses erste dreidimensionale anatomische Modell in einem gleichen räumlichen Bezugsrahmen wie das dreidimensionale Bild befindet,
- während einer Interventionsphase (P₂)
- Aufnahme (54) eines oder mehrerer präoperativer zweidimensionaler Bilder, die die vaskuläre Zielstruktur des Patienten, abgedunkelt oder nicht, umfasst,
- Schätzung (56) einer starren Transformation zwischen dem präoperativen dreidimensionalen Bild und den präoperativen zweidimensionalen Bildern,
- Schätzung (58) einer elastischen Transformation zwischen dem dreidimensionalen Bild und den zweidimensionalen Bildern in Abhängigkeit von der starren Transformation und einer Simulation von vaskulären Deformationen, die durch eine Einführung des Werkzeugs in die vaskuläre Zielstruktur induziert werden, wobei die Simulation in Abhängigkeit von einem lokalen Zustand der vaskulären Zielstruktur, einer Relation zwischen der vaskulären Zielstruktur und ihrer direkten Umgebung und von mechanischen Eigenschaften des endovaskulären Werkzeugs erhalten wird,
- Anwendung (60) einer Kombination der starren Transformation und der elastischen Transformation an dem ersten dreidimensionalen anatomischen Modell (M₁), das für den Patienten spezifisch ist, um ein zweites dreidimensionales Modell (M₂), das für den Patienten spezifisch ist, zu erhalten,
- Anzeige (62) des zweiten dreidimensionalen Modells, das für den Patienten spezifisch ist, in Überlagerung über die aufgenommenen zweidimensionalen Bilder.

## Claims

1. System for helping to guide an endovascular tool in vascular structures, comprising an imaging device able to acquire two-dimensional images of portions of the body of a patient, a programmable device and a viewing unit, said system being **characterised in that** it is adapted for:
- during an initial planning phase (P₁),
- - obtaining and memorising (50) a preoperative three-dimensional image comprising a target vascular structure of a patient,
- - determining (52) a first three-dimensional anatomical model (M₁) specific to the patient from the three-dimensional image acquired, this first three-dimensional anatomical model (M₁) being located in the same spatial reference as the three-dimensional image,
- during an intervention phase (P₂),
- - acquiring (54) one or several perioperative two-dimensional images comprising the target vascular structure of the patient, opacified or not,
- - estimating (56) a rigid transformation between the preoperative three-dimensional image and the perioperative two-dimensional images, said system being **characterised in that** it is further adapted for:
- - estimating (58) an elastic transformation between said three-dimensional image and the two-dimensional images according to the rigid transformation and a simulation of vascular deformations induced by introducing the tool into the target vascular structure, said simulation being obtained as a function of a local state of the target vascular structure, a relationship between the target vascular structure and its direct environment, and mechanical properties of the endovascular tool,
- - applying (60) a combination of the rigid transformation and of the elastic transformation to the first three-dimensional anatomical model (M₁) specific to the patient in order to obtain a second three-dimensional model (M₂) specific to the patient,
- - displaying (62) on the viewing unit said second three-dimensional model specific to the patient in superposition on the two-dimensional images acquired.

2. System according to claim 1, wherein the system is adapted for modelling (64) vascular structures of the patient from the preoperative three-dimensional image acquired during the estimating (58) of an elastic transformation.

3. System according to one of claims 1 or 2, wherein the system is adapted for constructing (66) a biomechanical interaction model between the vascular structures and the endovascular tool during the estimating (58) of an elastic transformation.

4. System according to claim 3, wherein the system is adapted for performing a simulation (68, 70) of the interaction between the endovascular tool and the target vascular structure by using a finite element analysis method.

5. System according to any of claims 1 to 4, wherein the system is further adapted for implementing a correction using said perioperative two-dimensional images during the estimating (58) of the elastic transformation.

6. System according to claim 5, wherein the system is further adapted for performing, during said correction, a projection of simulated positions of the endovascular tool on at least one perioperative two-dimensional image taken after the actual introduction of the endovascular tool, and a quantification of the difference between said simulated positions and actual positions of the endovascular tool.

7. System according to claim 6, wherein the system is further adapted for performing a recalibration between said simulated positions and said actual positions.

8. System according to any of claims 1 to 7, wherein the first three-dimensional anatomical model specific to the patient is represented by a structure among a volume, a mesh of points, a set of contours and a set of anatomical markers.

9. System according to any of claims 1 to 8, wherein the estimating of a rigid transformation is carried out by automatic, semi-automatic or manual recalibration, between the preoperative three-dimensional image and at least one perioperative two-dimensional image.

10. Computer program product comprising instructions for implementing the following steps of a method for helping to guide an endovascular tool in vascular structures during the execution of the program by a processor of a programmable device:
- during an initial planning phase (P₁),
- - obtaining and memorising (50) a preoperative three-dimensional image comprising a target vascular structure of a patient,
- - determining (52) a first three-dimensional anatomical model (M₁) specific to the patient from the three-dimensional image acquired, this first three-dimensional anatomical model (M₁) being located in the same spatial reference as the three-dimensional image,
- during an intervention phase (P₂),
- - acquiring (54) one or several perioperative two-dimensional images comprising the target vascular structure of the patient, opacified or not,
- - estimating (56) a rigid transformation between the preoperative three-dimensional image and the perioperative two-dimensional images,
- - estimating (58) an elastic transformation between said three-dimensional image and the two-dimensional images according to the rigid transformation and a simulation of vascular deformations induced by introducing the tool into the target vascular structure, said simulation being obtained as a function of a local state of the target vascular structure, a relationship between the target vascular structure and its direct environment, and mechanical properties of the endovascular tool,
- - applying (60) a combination of the rigid transformation and of the elastic transformation to the first three-dimensional anatomical model (M₁) specific to the patient in order to obtain a second three-dimensional model (M₂) specific to the patient,
- - displaying (62) on the viewing unit said second three-dimensional model specific to the patient in superposition on the two-dimensional images acquired.
